# EUROPEAN PATENT SPECIFICATION

(11) **EP 4 266 988 B1**
(45) Date of publication and mention of the grant of the patent: **03.06.2026**
(21) Application number: 21911749.6
(22) Date of filing: 24.12.2021
(51) Int. Cl.: A61B 5/00, G16H 40/00, A61B 5/11, A61B 5/22

(54) **WEARABLE ROBOTIC DEVICE**
TRAGBARE ROBOTISCHE VORRICHTUNG
DISPOSITIF ROBOTISÉ À PORTER

(30) Priority: 25.12.2020 TR 202021740
(43) Date of publication of application: 01.11.2023
(73) Proprietor: Istanbul Medipol Universitesi, 34810 Istanbul (TR)
(72) Inventor: HOCAOGLU, Elif, 34810 Beykoz / Istanbul (TR)
(74) Representative: Simsek, Meliha Merve
(86) International application number: PCT/TR2021/051495
(87) International publication number: WO 2022/139772

(56) References cited:
- WO-A1-2014/186537
- WO-A1-2020/087844
- US-A1- 2014 074 179
- US-A1- 2018 064 401
- US-A1- 2020 289 814
- US-A1- 2021 333 877
- US-B1- 10 085 689
- ISMAIL HAKAN ERTAS: "AssistON-FINGER: An under-actuated finger exoskeleton for robot-assisted tendon therapy", ROBOTICA : AN OFFICIAL JOURNAL OF THE INTERNATIONAL FEDERATION OF ROBOTICS, vol. 32, no. 8, 17 July 2014 (2014-07-17), GB , pages 1363 - 1382, XP009538613, ISSN: 0263-5747, DOI: 10.1017/S0263574714001957

## Description

### TECHNICAL FIELD

The invention of the application relates to a wearable robotic device that can replace the standard clinical tests used in the follow-up of MS (Multiple Sclerosis) disease, can make measurements with high precision, and enables monitoring the phase of MS disease and the process of response to the treatment at frequent intervals.

The invention also allows the patients to perform dynamic activities and contributes to the good course of the disease as it is able to stimulate when the patients deliver different performance than the healthy people normals.

### THE STATE OF THE ART

Multiple sclerosis is an autoimmune disease that affects the brain and spinal cord. It is referred to as MS for short. It is the attempt of defensive cells of immune system to destroy the myelin sheath (it can be called as a kind of fatty membrane layer) around nerve cells (neurons) by perceiving as a foreign antigen to the body for an unknown reason. Today, there are standard clinical tests with various features, each of which has its own assessment scales, which are run in cooperation with neurologists and physiotherapists in the follow-up process of MS patients and used to observe the deterioration in the upper/lower extremity functions of the patients. In these commonly used tests, the level of deterioration in the limb functions of patients is evaluated by a physiotherapist according to the patient's performance during the desired activity, and information about the level of the disease is obtained according to the score he/she receives. However, non-objective scoring, which depends on the opinion of the physiotherapist, is open to erroneous evaluations and may differ from person to person. Also, the said clinical tests cannot provide detailed information about the functional disorder of the patient. Unfortunately, since an objective evaluation system has not yet been implemented in our country and all over the world, the course of the disease is still provided by an evaluation system based on human observation in such a neurologically based disability, whose follow-up is of vital importance, such as MS, and the treatment method is determined based on this evaluation.

One of the prior art documents is US 10085689 where a device and method for monitoring and assessment of movement disorder symptoms is disclosed. The document also mentions that the symptom of diseases such as MS or Parkinson's fluctuate with respect to time and relative to certain conditions such as stress and other psychological factors and this makes it difficult of physicians to make an accurate assessment of the patient. Thus, the inventors of US 10085689 disclose a device which allows for remote monitoring of the patient.

Another prior art document is WO 2014/186537, discloses a game based sensorimotor rehabilitator which lets the individuals to interact with functional objects to regain lost skills. In a way, the disclosure of WO 2014/186537 introduces the patients with a method for self-therapy by forming alternative neural pthways to overcome the damage produced by various diseases such as stroke.

Additionally, WO 2020/087844 discloses a flexible finger-wearable tactile pressure feedback device which comprises a fingertip cover that is worn on the distal phalanx of a finger, a middle finger band worn on a proximal phalanx of a finger and an elastically bendable outer transmission rod and an elastically bendable inner transmission rod. The invention disclosed in said document is easy to wear and allows easy movement of the fingers while providing tactile pressure feedback information to the fingers.

The treatment methods available today include basically, drug treatments recommended by neurologists against the exacerbations/relapses of existing symptoms in MS disease or the presence of new symptoms, rehabilitation therapies in order to maintain or improve the functions of the patient, supportive alternative medicine methods such as vitamins, exercise, diet and acupuncture. Although the mentioned methods have an important role in the treatment of the disease, they require the patient to visit hospitals and rehabilitation centers permanently and to need professional support such as doctors and therapists.

### BRIEF DESCRIPTION OF THE INVENTION

The present invention relates to a wearable robotic device that can provide an objective evaluation with the features to solve the above-mentioned problems, can be used easily and is suitable for follow-up of the patient at frequent intervals.

Since the treatment method of MS has not been determined yet, the follow-up of each method applied is of great importance in terms of observing the response of the disease to the method. For this reason, frequent follow-up of the patient is important for determining the most appropriate treatment method. Under these circumstances, the patient often needs to undergo various clinical tests accompanied by a physiotherapist, and the results must be discussed with the neurologist. This situation causes a great burden for the patient, both in terms of economy and time, and sometimes patients have difficulty in carrying out these follow-ups with a regular program. In tests performed by traditional physiotherapists, the patient's performance is evaluated based on a single parameter. The patient has to be subjected to a large number of tests for handling the evaluations from multiple perspectives. At this point, by means of the bio-mechatronic device we have proposed,
- The patient's data is recorded in real time at defined time intervals for each activity.
- Multiple variable parameters of the patient are evaluated in a single experiment.
- The patient has the opportunity to perform similar activities that he/she frequently performs in daily life, and thus, to measure the accuracy and performance of similar dynamic movements under the supervision of the doctor.

The wearable bio-mechatronic device mentioned in the invention provides the possibilities that the patient can easily carry from one place to another, it can be easily used by patients of all age groups, and that he/she can easily share the test results with their doctor over the internet when necessary. Most importantly, the control of patient will be provided at frequent intervals, and the effect and accuracy of the treatment method will be evaluated.

In the wearable robotic device developed with this invention, it is aimed that;
- The phase of MS disease and response to treatment are monitored at frequent intervals,
- The objective measurements are presented about the course of MS disease,
- It can monitor MS disease by easily carrying it to any environment where the patient is present (easy portability),
- Patients of all age groups can easily use the device,
- By means of the frequent follow-up of the patient, feedback on the accuracy of the treatment method can be provided,
- The patient receives warnings depending on their performance and is encouraged to perform the given tasks more accurately
- The tasks similar to daily activities can be assigned to the patient,
- The patient is allowed to use hand functions under static and dynamic conditions,
- The status of the patient can be evaluated through various performance metrics such as the success of the patient to hold/release an object without sliding, and the comparison of the force applied against the sliding according to the required value (according to the ratio of force components applied by a healthy individual),
- Due to the electromechanical structure of the proposed rehabilitation and clinical evaluation system, the evaluation is provided in an objective way, away from the individual point of view,
- Valuable supports are provided to the treatment plan proposed by the doctor, with objective evaluations about the patient's condition.

### DEFINITIONS OF THE FIGURES DESCRIBING THE INVENTION

The figures used to better explain the wearable robotic device developed with this invention are given below.

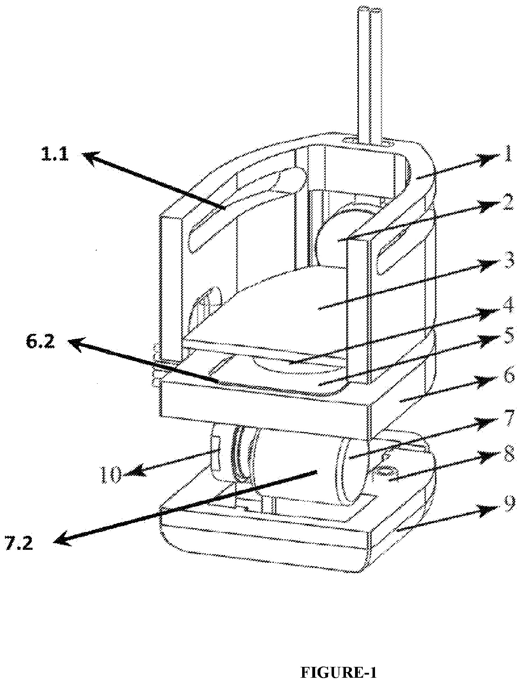

Figure 1 - A perspective view of placement of the part of the solid model of the wearable device

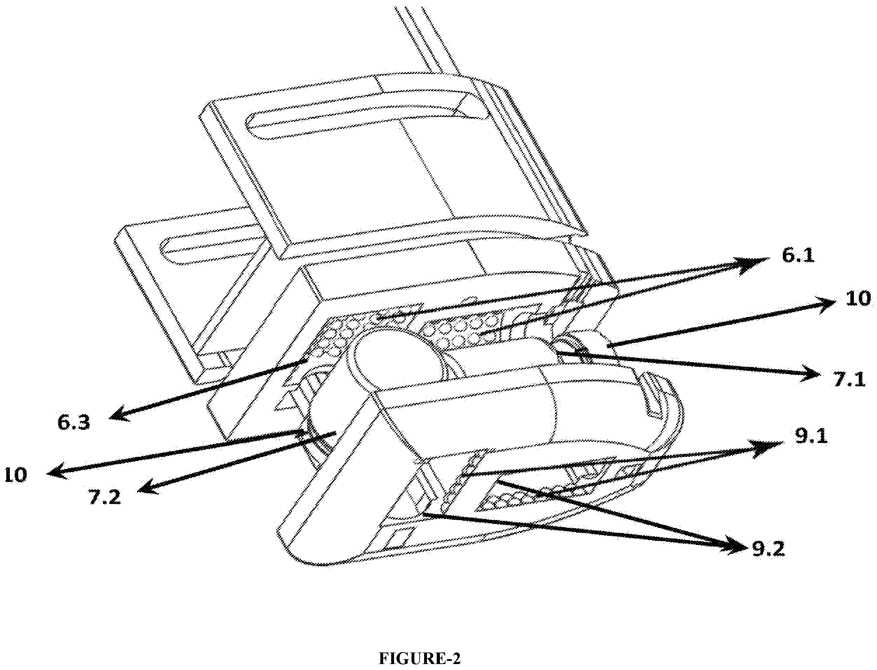

Figure 2 - A perspective view of placement of the part of the solid model of the wearable device from another angle

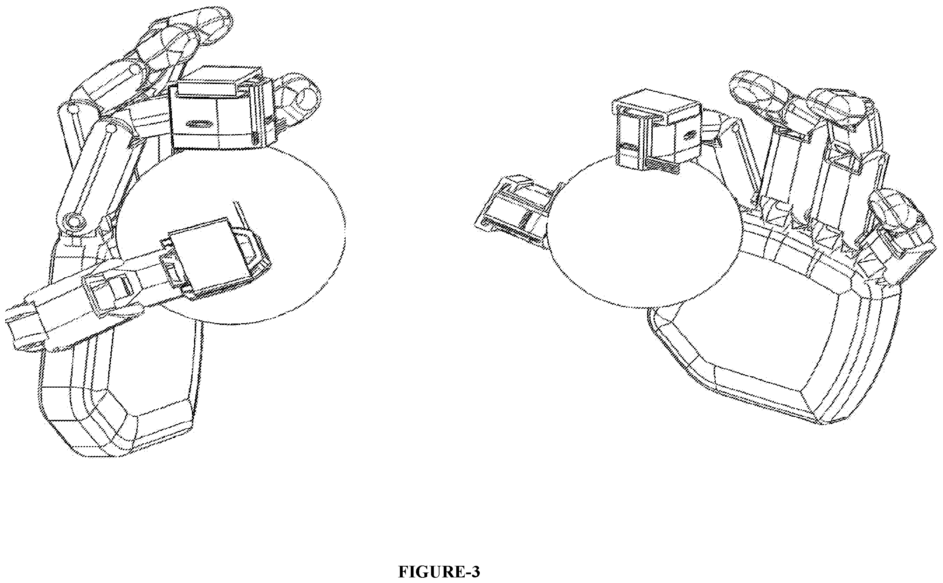

Figure 3 - A view showing the object holding success of the fingertip wearable device

### DEFINITIONS OF ELEMENTS AND PARTS CONSTITUTING THE INVENTION

The parts and elements of the wearable robotic device developed with this invention are individually numbered and listed below.
**1.** Upper Part
   **1.1.** Fixing Openings
**2.** Vibration Motor
**3.** Finger Protector
**4.** Force Focuser
**5.** Force Sensing Resistor
**6.** Intermediate Part
   **6.1.** Hemispherical Textured Upper Surfaces
   **6.2.** Slot
   **6.3.** Semi-Cylinder Slot
**7.** Cylindrical Structure with Magnetic Part
   **7.1.** Vertical Slip Sensor
   **7.2.** Horizontal Slip Sensor
**8.** Screw
**9.** Lower Part
   **9.1.** Hemispherical Textured Lower Surfaces
   **9.2.** Semi-Cylinderical Slot
**10.** Magnetic Rotary Encoder

### DETAILED DESCRIPTION OF THE INVENTION

The invention relates to a wearable robotic device that can replace the standard clinical tests used in the follow-up of MS (Multiple Sclerosis) disease, can make measurements with high precision, and enables monitoring the phase of MS disease and the process of response to the treatment at frequent intervals. The invention also contributes to the good course of the disease, as it can give a warning if the patient performs his dynamic activities and presents a different performance compared to the normal of healthy people. Due to the feature of the design that emphasizes that the patient can move an object from one point to another using two fingers, place the object in a space with appropriate geometry by making manipulative movements, hold objects of different weights, in other words hand functions that require skill, it covers tasks in the pinch grasp category.

To increase interaction and manipulation while performing tasks, a wearable fingertip robotic device with sufficient slots for the distal phalanges (bones at the most extreme tips of the fingers) has been developed that can sense the initial moment of sliding in various directions and force change. After the said robotic device is attached to the fingertip, it can be fixed to the finger with touch and close fasteners.

The mechanical design of the invention mainly consists of two layers with integrated sensors presented as exploded view in Figure-1 and Figure-2. Lightweight design criterion is met by the selection of flexible resin material with density of 1.1 g / cm³, by avoiding the size from being larger than the required for the fingertips (18x27x11mm), and by designing the overall platform durable yet thin enough. In another embodiment of the invention, a flexible resin material with a lower density can be selected.

To meet the size and shape criteria, the two main bodies of the wearable device are produced with UV LCD 3d printer production technique, which is functional for the needs of users of all sizes. On the other hand, ergonomics criterion is realized with a wearable device designed as minimally as possible, which allows to keep the working space of the fingertip as wide as possible.

According to the sensory functionality criterion, force sensing resistors (5) are placed in their slots (6.2) in the mechanical design of the wearable device to sense the force in the direction of pressure. These sensors (5), which can detect a maximum force of 44N, can also measure the upper limit of a person who produces a maximum force of 12N with his/her fingertip. The slip sensation is achieved by detecting its nearly stationary motion (of the cylindrical rotary parts (7.1 and 7.2) placed between the intermediate part (6) and the lower part (9) by 12-bit non-contact magnetic rotary encoders (10) that are sensitive enough to detect motion) (1 count corresponds to 0,09° revolution). The device also gives warnings according to the over or sub-normal performance during the activities performed by the patient by means of the button-type shaft-less mini vibration motor (2), which is placed to coincide with the nose of the finger. Thus, a manual activity helps to repair the diseased area in the neural circuit. It is desired to measure whether the success of MS patients in holding objects is worse than healthy people, so whether the force applied by the fingers to the object is sufficient or if more force is applied to prevent the object from falling. In case of applying less force to the object, vibrational warning is given to the patient by means of the vibration motor (2) at the moment of detection of instant minimal slip.

It is important for people to be able to easily perform the tasks given during the experiment, especially dynamic activities, not to be restricted by any mechanical obstacle during the activity, to provide a harmonious hand-eye coordination and to subject their concentration to the experiment only in terms of accurate evaluation and follow-up. For this reason, the communication of the system is provided via a microcontroller card which allows the data transfer to be transferred by wireless communication and is connected to the human wrist.

In an embodiment of the invention, the data collected with the wireless internet connection module (Wi-fi Module) in the device can be periodically sent to the patient's doctor.

In its most basic form, a wearable robotic device that can make measurements with high precision to replace the standard clinical tests used in the follow-up of MS (Multiple Sclerosis) disease, enables monitoring the phase of MS disease and the process of response to the treatment at frequent intervals includes
- An upper part (1), which has a "U" shaped structure and has a sufficient slot for the most extreme bones of the patient's fingers,
- A vibration motor (2), which coincides with the nose of the finger after it is placed on the upper part, and adapted to help the repair of the diseased area in the neural circuit of a manual activity by giving warnings according to the over or sub-normal performance during the activities performed by the patient,
- A Finger protector (3), in which the lower end of the finger touches and which is configured to prevent damage to both the finger and a force sensing resistor (5) by preventing the finger from coming into direct contact with the force sensing resistor (5),
- A force focuser (4), which focuses the force applied by the patient while holding an object,
- Force sensing resistors (5) that sense the force in the pressure direction,
- An intermediate part (6) on which the upper part (1), the finger protector (3), the force focuser (4) and the force sensing resistor (5) are placed and which is one of the elements forming the main body,
- A lower part (9), which is another of the elements that form the main body,
- A vertical slip sensor (7.1) and a horizontal slip sensor (7.2) with a cylindrical structure (7) with magnetic parts located between the intermediate part (6) and the lower part (9),
- Magnetic rotary encoders (10) that allow detecting the movement of the vertical slip sensor (7.1) and the horizontal slip sensor (7.2), and
- A microcontroller card that allows data transfer to be transferred via wireless communication and is worn on the wrist like a watch.

Since the upper part has a "U" shaped structure, the device is fixed to the finger by passing velcro bands through the fixing openings (1.1) on the upper part in order to fix the finger to the device after the finger is placed on the upper part.

On the upper surface (6) of the intermediate part facing the lower face of the finger, there is a slot (6.2) into which the force sensing resistor (5) is fixed. On the lower surface (6) of the intermediate part, there is a semi-cylindrical slot (6.3) into which half of the vertical slip sensor (7.1) and the horizontal slip sensor (7.2) can be inserted. The surfaces of the semi-cylindrical slot (6.3) have hemispherical textured lower surfaces (6.1). The hemispherical textured lower surfaces (6.1) provide a balanced rotation of the cylindrical structures (7) with magnetic parts. The hemispherical textured lower surfaces (6.1) also provide less friction between the two surfaces of the cylindrical structures with magnetic parts (7) and the semi-cylindrical slots (6.3). Thus, the cylindrical structures (7) with magnetic parts are provided to rotate more easily without being exposed to frictional resistance. This enables the starting point of the slip to be detected precisely.

On the surface of the lower part (9) facing the intermediate part (6), there is a semi-cylindrical slot (9.2) into which half of the vertical slip sensor (7.1) and the horizontal slip sensor (7.2) can be inserted. The surfaces of the semi-cylindrical slot (9.2) have hemispherical textured lower surfaces (9.1). The hemispherical textured lower surfaces (9.1) provide a balanced rotation of the cylindrical structures (7) with magnetic parts.

When the semi-cylindrical slot on the lower part (9) and the semi-cylindrical slots on the intermediate part (6) are overlapped, they completely surround the cylindrical structures with magnetic parts. The connection of the lower part (9) and the intermediate part (6) is made by means of the screws (8). The screws (8) providing the connection of the lower part (9) and the intermediate part (6) are at least two.

## Claims

1. A wearable robotic device adapted to make measurements with high precision to replace the standard clinical tests used in the follow-up of MS (Multiple Sclerosis) disease, enables monitoring the phase of MS disease and the process of response to the treatment at frequent intervals, wherein, in its most basic form, the wearable robotic device includes
• An upper part (1), which has a "U" shaped structure and has a sufficient slot for distal phalanx of the patient's fingers,
• A vibration motor (2), which corresponds to the finger tip after inserting it into the upper part, adapted to help recover the diseased area in the neural loop during an activity performed by hand by stimulating based on the over or subnormal performance of the patient during the activities,
• A finger isolator (3), which the lower end of the finger is in contact, configured to hinder damage to both the finger and a force sensing resistor (5) by preventing the finger directly contact the force sensing resistor (5), a force focuser (4), which focuses the force applied by the patient while holding an object,
• Force sensing resistors (5) that sense the force in the pressure direction,
• An intermediate part (6) on which the upper part (1), the finger protector (3), the force focuser (4) and the force sensing resistor (5) are placed and which is one of the elements forming the main body,
• A lower part (9), which is another of the elements that form the main body,
• A vertical slip sensor (7.1) and a horizontal slip sensor (7.2) have a cylindrical structure (7) with magnetic parts located between the intermediate part (6) and the lower part (9),
• Magnetic rotary encoders (10) that allow detecting the movement of the vertical slip sensor (7.1) and the horizontal slip sensor (7.2),
• A microcontroller card that allows to transmit data by wireless communication and is worn on a wrist.

2. A wearable robotic device as in claim 1, **characterized in that;** it includes the fixing slots located on the upper part (1) that allows the finger to be fixed to the device.

3. A wearable robotic device as in claim 1, **characterized in that;** it includes a slot (6.2) into which the force-sensing resistor (5) is fixed on the upper surface (6) of the intermediate part, facing the lower face of the finger.

4. A wearable robotic device as in claim 1, **characterized in that;** it includes a semi-cylindrical slot (6.3) into which half of the vertical slip sensor (7.1) and the horizontal slip sensor (7.2) can be inserted on the lower surface of the intermediate part (6).

5. A wearable robotic device as in claim 4, **characterized in that;** the semi-cylindrical slot (6.3) has spherical textured upper surfaces (6.1) that ensure stable rotation of the cylindrical structures with magnetic parts (7) and provide less friction between the cylindrical structures with magnetic parts (7) and the surfaces of the semi-cylindrical slots (6.3).

6. A wearable robotic device as in claim 1, **characterized in that;** it includes a semi-cylindrical slot (9.2) into which half of the vertical slip sensor (7.1) and the horizontal slip sensor (7.2) can be inserted on the upper surface of the lower part (9).

7. A wearable robotic device as in claim 6, **characterized in that;** the semi-cylinder slot (9.2) has hemispherical textured upper surfaces (9.1) that ensure stable rotation of the cylindrical structures with magnetic parts (7).

8. A wearable robotic device as in any one of the preceding claims, **characterized in that;** it has at least two screws (8) that provide the connection of the lower part (9) and the intermediate part (6).

9. A wearable robotic device as in any of the preceding claims, **characterized in that;** it has an internet connection module that enables the data collected from the patient to be transmitted to the patient's doctor periodically.

10. A wearable robotic device as in any one of the preceding claims, **characterized in that;** the upper part (1), the intermediate part (6) and the lower part (9) are produced from a flexible resin material.

11. A robotic device as in claim 10, **characterized in that;** the upper part (1), the intermediate part (6) and the lower part (9) are produced from a flexible resin material with a density of 1.1 g/cm3.

## Patentansprüche

1. Eine Tragbare Robotervorrichtung, das für hochpräzise Messungen ausgelegt ist und die bei der Nachsorge von MS (Multiple Sklerose) verwendeten klinischen Standardtests ersetzt, ermöglicht die Überwachung des MS-Krankheitsstadiums und des Ansprechens auf die Behandlung in kurzen Abständen, **dadurch gekennzeichnet, dass** es in seiner grundlegendsten Form Folgendes umfasst:
• Ein Oberteil (1), das eine "U" -förmige Struktur aufweist und einen ausreichenden Schlitz für die distale Phalanx der Finger des Patienten aufweist,
• Einen Vibrationsmotor (2), der an der Fingerspitze angebracht ist, nachdem er in den oberen Teil eingeführt wurde und angepasst wurde, um den erkrankten Bereich in der Nervenschleife während einer von Hand ausgeführten Aktivität wiederherzustellen, indem er den Patienten basierend auf seiner übermäßigen oder unterdurchschnittlichen Leistung während der Aktivität stimuliert,
• Ein Fingerisolator (3), mit dem das untere Ende des Fingers in Kontakt steht, der so konfiguriert ist, dass er Schäden sowohl am Finger als auch an einem Kraftmesswiderstand (5) verhindert, indem er den direkten Kontakt des Fingers mit dem Kraftmesswiderstand (5) verhindert, ein Kraftfokussierer (4), der die vom Patienten beim Halten eines Objekts ausgeübte Kraft fokussiert,
• Kraftmesswiderstände (5), die die Kraft in Druckrichtung erfassen,
• Ein Zwischenteil (6), auf dem das Oberteil (1), der Fingerschutz (3), der Kraftfokussierer (4) und der Kraftmesswiderstand (5) angeordnet sind und das eines der Elemente ist, die den Hauptkörper bilden,
• Ein unterer Teil (9), der ein weiteres Element des Hauptkörpers bildet,
• Ein vertikaler Schlupfsensor (7.1) und ein horizontaler Schlupfsensor (7.2) weisen eine zylindrische Struktur (7) mit magnetischen Teilen auf, die sich zwischen dem Zwischenteil (6) und dem Unterteil (9) befinden,
• Magnetische Drehgeber (10), die eine Erfassung der Bewegung des vertikalen Schlupfsensors (7.1) und des horizontalen Schlupfsensors (7.2) ermöglichen,
• Eine Mikrocontroller-Karte, die die Übertragung von Daten über drahtlose Kommunikation ermöglicht und am Handgelenk getragen wird.

2. Eine Tragbare Robotervorrichtung nach Anspruch 1, **dadurch gekennzeichnet, dass** es Befestigungsschlitze am Oberteil (1) mit denen der Finger an der Vorrichtung befestigt werden kann.

3. Eine Tragbare Robotervorrichtung nach Anspruch 1, **dadurch gekennzeichnet, dass** es einen Schlitz (6.2) umfasst, in dem der Kraftmesswiderstand (5) auf der Oberfläche (6) des Zwischenteils befestigt ist, die der Unterseite des Fingers zugewandt ist.

4. Eine Tragbare Robotervorrichtung nach Anspruch 1, **dadurch gekennzeichnet, dass** es einen halbzylindrischen Schlitz (6.3) umfasst, in den die Hälfte des vertikalen Schlupfsensors (7.1) und des horizontalen Schlupfsensors (7.2) auf der Unterseite des Zwischenteils (6) eingeführt werden kann.

5. Eine Tragbare Robotervorrichtung nach Anspruch 4, **dadurch gekennzeichnet, dass** der halbzylindrische Schlitz (6.3) kugelförmig strukturierte Oberflächen (6.1) aufweist, die eine stabile Drehung der zylindrischen Strukturen mit magnetischen Teilen (7) gewährleisten und für weniger Reibung zwischen den zylindrischen Strukturen mit magnetischen Teilen (7) und den Oberflächen der halbzylindrischen Schlitze (6.3) sorgen.

6. Eine Tragbare Robotervorrichtung nach Anspruch 1, **dadurch gekennzeichnet, dass** es einen halbzylindrischen Schlitz (9.2) umfasst, in den die Hälfte des vertikalen Schlupfsensors (7.1) und des horizontalen Schlupfsensors (7.2) auf der Oberseite des unteren Teils (9) eingesetzt werden kann.

7. Eine Tragbare Robotervorrichtung nach Anspruch 6, **dadurch gekennzeichnet, dass** der Halbzylinderschlitz (9.2) halbkugelförmig strukturierte strukturierte Oberflächen (9.1) aufweist, die eine stabile Drehung der zylindrischen Strukturen mit magnetischen Teilen (7) gewährleisten.

8. Eine Tragbare Robotervorrichtung nach einem der vorhergehenden Ansprüche, **dadurch gekennzeichnet, dass** es mindestens zwei Schrauben (8) aufweist, die die Verbindung des Unterteils (9) und des Zwischenteils (6) bereitstellen.

9. Eine Tragbare Robotervorrichtung nach einem der vorhergehenden Ansprüche, **dadurch gekennzeichnet, dass** es über ein Internetverbindungsmodul verfügt, das es ermöglicht, die vom Patienten gesammelten Daten periodisch an den Arzt des Patienten zu übertragen.

10. Eine Tragbare Robotervorrichtung nach einem der vorhergehenden Ansprüche, **dadurch gekennzeichnet, dass** das Oberteil (1), das Zwischenteil (6) und das Unterteil (9) aus einem flexiblen Kunststoffmaterial hergestellt sind.

11. Eine Robotervorrichtung nach Anspruch 10, **dadurch gekennzeichnet, dass** das Oberteil (1), das Zwischenteil (6) und das Unterteil (9) aus einem flexiblen Kunststoffmaterial mit einer Dichte von 1.1 g/cm3 hergestellt sind.

## Revendications

1. Dispositif robotique portable adapté pour effectuer des mesures de haute précision afin de remplacer les tests cliniques standard utilisés dans le suivi de la SEP (sclérose en plaques), permettant de surveiller la phase de la SEP et le processus de réponse au traitement à intervalles fréquents, **caractérisé en ce que,** dans sa forme la plus basique, il comprend
• Une partie supérieure (1), qui présente une structure en forme de « U » et une fente suffisante pour la phalange distale des doigts du patient,
• Un moteur vibrant (2), qui correspond à l'extrémité du doigt après l'avoir inséré dans la partie supérieure, adapté pour aider à récupérer la zone malade dans la boucle neuronale pendant une activité effectuée à la main en stimulant en fonction des performances supérieures ou inférieures à la normale du patient pendant les activités,
• Un isolateur de doigt (3), auquel l'extrémité inférieure du doigt est en contact, configuré pour empêcher les dommages à la fois au doigt et à une résistance de détection de force (5) en empêchant le doigt d'entrer en contact direct avec la résistance de détection de force (5), un concentrateur de force (4) qui concentre la force appliquée par le patient lorsqu'il tient un objet,
• Résistances de détection de force (5) qui détectent la force dans le sens de la pression,
• Une partie intermédiaire (6) sur laquelle sont placés la partie supérieure (1), le protecteur de doigt (3), le concentrateur de force (4) et la résistance de détection de force (5) et qui est l'un des éléments formant le corps principal,
• Une partie inférieure (9), qui est un autre des éléments formant le corps principal,
• Un capteur de glissement vertical (7.1) et un capteur de glissement horizontal (7.2) qui présentent une structure cylindrique (7) avec des parties magnétiques situées entre la partie intermédiaire (6) et la partie inférieure (9),
• Codeurs rotatifs magnétiques (10) qui permettent de détecter le mouvement du capteur de glissement vertical (7.1) et du capteur de glissement horizontal (7.2),
• Une carte à microcontrôleur qui permet de transmettre des données par communication sans fil et qui se porte au poignet.

2. Dispositif robotique portable selon la revendication 1, **caractérisé en ce qu'**il comprend les fentes de fixation situées sur la partie supérieure (1) qui permettent de fixer le doigt au dispositif.

3. Dispositif robotique portable selon la revendication 1, **caractérisé en ce qu'**il comprend une fente (6.2) dans laquelle la résistance de détection de force (5) est fixée sur la surface supérieure (6) de la partie intermédiaire, faisant face à la face inférieure du doigt.

4. Dispositif robotique portable selon la revendication 1, **caractérisé en ce qu'**il comprend une fente semi-cylindrique (6.3) dans laquelle la moitié du capteur de glissement vertical (7.1) et le capteur de glissement horizontal (7.2) peuvent être insérés sur la surface inférieure de la partie intermédiaire (6).

5. Dispositif robotique portable selon la revendication 4, **caractérisé en ce que** la fente semi-cylindrique (6.3) présente des surfaces supérieures texturées sphériques (6.1) qui assurent une rotation stable des structures cylindriques avec des parties magnétiques (7) et réduisent le frottement entre les structures cylindriques avec des parties magnétiques (7) et les surfaces des fentes semi-cylindriques (6.3).

6. Dispositif robotique portable selon la revendication 1, **caractérisé en ce qu'**il comprend une fente semi-cylindrique (9.2) dans laquelle la moitié du capteur de glissement vertical (7.1) et le capteur de glissement horizontal (7.2) peuvent être insérés sur la surface supérieure de la partie inférieure (9).

7. Dispositif robotique portable selon la revendication 6, **caractérisé en ce que** la fente semi-cylindrique (9.2) présente des surfaces supérieures texturées hémisphériques (9.1) qui assurent une rotation stable des structures cylindriques avec des parties magnétiques (7).

8. Dispositif robotique portable selon l'une quelconque des revendications, **caractérisé en ce qu'**il présente au moins deux vis (8) qui assurent la connexion entre la partie inférieure (9) et la partie intermédiaire (6).

9. Dispositif robotique portable selon l'une quelconque des revendications, **caractérisé en ce qu'**il présente un module de connexion internet qui permet aux données recueillies du patient d'être transmises périodiquement au médecin du patient.

10. Dispositif robotique portable selon l'une quelconque des revendications, **caractérisé en ce que** la partie supérieure (1), la partie intermédiaire (6) et la partie inférieure (9) sont fabriquées à partir d'un matériau en résine souple.

11. Dispositif robotique portable selon la revendication 10, **caractérisé en ce que** la partie supérieure (1), la partie intermédiaire (6) et la partie inférieure (9) sont fabriquées à partir d'un matériau en résine souple d'une densité de 1.1 g/cm3.
